# EUROPÄISCHE PATENTSCHRIFT

(11) **EP 1 337 210 B1**
(45) Veröffentlichungstag und Bekanntmachung des Hinweises auf die Patenterteilung: **30.11.2005**
(21) Anmeldenummer: 01998289.1
(22) Anmeldetag: 03.12.2001
(51) Int. Cl.: A61F 9/00

(54) **LÖSBARE IMPLANTATHALTERUNG**
REMOVABLE IMPLANT FIXING DEVICE
FIXATION D'IMPLANT LIBERABLE

(30) Priorität: 01.12.2000 DE 10060029
(43) Veröffentlichungstag der Anmeldung: 27.08.2003
(73) Patentinhaber: TD Verwaltungs GmbH, 90478 Nürnberg (DE)
(72) Erfinder: ECKMILLER, Rolf, 41460 Neuss (DE)
(74) Vertreter: Graf von Stosch, Andreas
(86) Internationale Anmeldenummer: PCT/EP2001/014077
(87) Internationale Veröffentlichungsnummer: WO 2002/043631

(56) Entgegenhaltungen:
- WO-A-00/40161
- WO-A-01/83025
- WO-A-85/03857
- WO-A-87/06117
- US-A- 5 935 155

## Beschreibung

Die vorliegende Erfindung betrifft eine Verankerung für implantierbare Mikrokontaktfolien als Neuroimplantate, insbesondere Retinaimplantate mit den Merkmalen des Oberbegriffs des Anspruchs 1.

Derartige Verankerungen sind beispielsweise aus der Druckschrift WO00/40161 bekannt. Eine Beschreibung eines Retinaimplantats, das mit den bekannten Verankerungsvorrichtungen im Auge befestigt wird, ist in dem Artikel "Development of flexible stimulation devices for a retina implant system" von Stieglitz et al., erschienen in Proceedings, 19^{th} International Conference - IEEE/EMBS, October 30 - November 2, 1997, Chicago, IL, USA, Seite 2.307 - 2.310 dargestellt.

Nach dem Stand der Technik werden Mikrokontaktfolien für Neuroprothesen, insbesondere die erwähnten Retinaimplantate als Sehprothesen mit Mikronägeln (micro tacks) epiretinal an der Netzhaut befestigt, indem diese Mikronägel durch Löcher in der Mikrokontaktfolie gesteckt und durch die Retina, das Pigmentepithel und die Aderhaut hindurch an der Sklera verankert werden. Dadurch sind diese Befestigungen nicht ohne nennenswerte Läsionen der Netzhaut wieder zu lösen, so dass eine Re-Explantation der Mikrokontaktfolie erheblich erschwert ist.

US-A-5,935,155 beschreibt eine als Retina-Implant vorgesehene Mikrokontaktfolie, welche mittels Mikronägeln ("tacks") an der Netzhaut eines Auges festlegbar ist. Die Mikronägel sind im wesentlichen rotationssymmetrisch ausgebildet. Ein Wechsel der Folie ist durch Entfernen der Nägel möglich. Für den Fall, dass ein Nagel die Folie nicht durchgreift, sondern nur randseitig überdeckt, kann ein Wechsel der Folie auch ohne Entfernen des Nagels vorgenommen werden. Eine alternative Festlegung besteht in Vernähung eines ersten Magneten mit der Netzhaut des Auges, wobei ein zweiter Magnet an der Folie angebracht ist, so dass die beiden Magneten lösbar aneinander festlegbar sind.

Es ist deshalb Aufgabe der vorliegenden Erfindung, eine lösbare Befestigung oder Halterung von Mikrokontaktfolien insbesondere an Retinagewebe zu schaffen.

Diese Aufgabe wird von einer Verankerung mit den Merkmalen des Anspruchs 1 gelöst.

Weil der Verankerungskopf die Implantatfläche zum Zwecke der Befestigung überragt und die Befestigung bei der Re-Explantation durch Hervorziehen der Implantatfolie unter dem Verankerungskopf gelöst werden kann, kann die Mikrokontaktfolie ohne eine zwangsläufige Beschädigung des darunter liegenden Gewebes entfernt werden. Es kann beispielsweise als Austausch für eine defekte Mikrokontaktfolie oder gegen ein im Wege der technischen Verbesserungen leistungsfähigeres Implantat eine neue Mikrokontaktfolie eingesetzt werden, ohne dass eine weitere Schädigung des Gewebes zu befürchten ist.

Wenn ein seitliches Hervorziehen der Mikrokontaktfolie unter den Verankerungsköpfen unerwünscht ist, kann der Verankerungskopf auch aus einem im Gewebe verbleibenden Grundkörper herausziehbar ausgeführt werden. Es kann weiter vorgesehen sein, dass der Verankerungskopf nach Art eines Knebelverschlusses schwenkbar gelagert ist und zur Halterung über die Oberseite der Mikrokontaktfolie geschwenkt werden kann. Zur Entfernung der Mikrokontaktfolie in einer zur Gewebeoberfläche normalen Richtung wird dann der Kopf derart verschwenkt, dass die Mikrokontaktfolie freigegeben wird. Schließlich kann für besondere Anwendungen auch vorgesehen sein, dass eine durch das Gewebe hindurchreichende Verankerungsstruktur im Gewebe befestigt ist, die einen lösbaren Verankerungskopf trägt, so dass dieser Verankerungskopf eingesetzt und entfernt werden kann, ohne das Gewebe zu beeinträchtigen.

Im folgenden werden Ausführungsbeispiele der vorliegenden Erfindung anhand der Zeichnung beschrieben. Es zeigen:
- Figur 1:: Einen Gewebeausschnitt aus der Retina mit einer Mikrokontaktfolie, die epiretinal auf dem Gewebeausschnitt aufliegt und die mit verschwenkbaren Verankerungsvorrichtungen befestigt ist;
- Figur 2:: die Verankerungsvorrichtung gemäß Figur 1 in einer vergrößerten Darstellung.

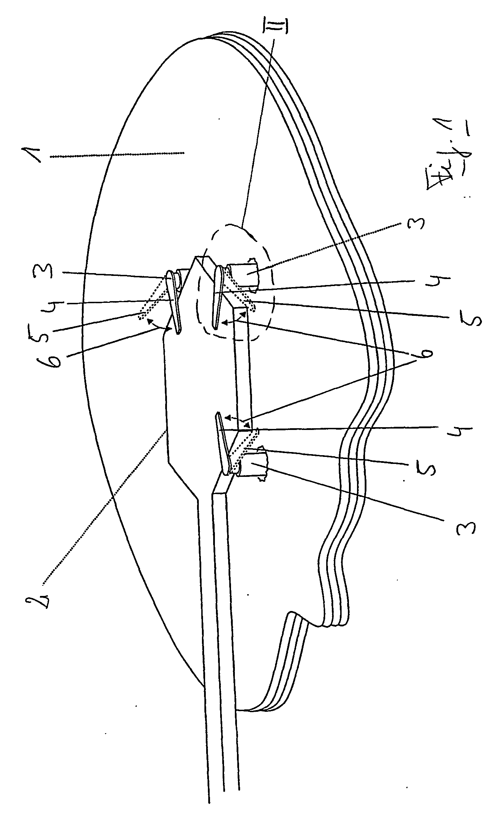

In der Figur 1 ist schematisch ein Ausschnitt aus einer Retina 1 dargestellt, die mit der der Linse zugewandten Seiten nach oben abgebildet ist. Eine Mikrokontaktfolie 2 liegt flächig auf der Oberfläche der Retina 1 auf. Die nicht sichtbaren Kontakte der Mikrokontaktfolie 2 sind der Retina 1 zugewandt, um dort in an sich bekannter Weise durch elektrische Impulse Sinneswahrnehmungen erzeugen zu können.

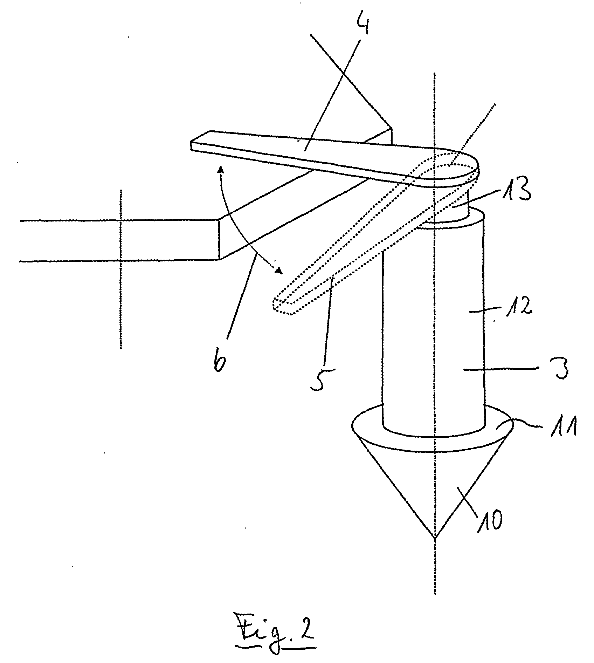

Die Mikrokontaktfolie 2 ist bei dieser Darstellung durch drei Verankerungsvorrichtungen 3 gesichert, die jeweils einen verschwenkbaren Hebel 4 als Verankerungskopf aufweisen. Die Hebel 4 sind in ihrer Verankerungsstellung dargestellt. Sie sind um eine senkrecht zu dem Implantat 2 orientierte Achse in eine Freigabestellung 5 verschwenkbar, was durch die Doppelpfeile 6 angedeutet ist. Der Ausschnitt II ist in der Figur 2 näher dargestellt.

Die Figur 2 zeigt die Verankerungsvorrichtung 3 in einer vergrößerten Darstellung, wobei zur Veranschaulichung die Retina 1 nicht dargestellt ist. Der Verankerungskopf 3 weist ähnlich den als micro tacks bekannten Mikronägeln eine kegelförmige Spitze 10 zum Eintreiben in die Retina auf, die an der der Spitze abgewandten Seite eine Planfläche 11 als Hinterschneidung trägt. Durch diese Gestaltung sitzt die Verankerungsvorrichtung 3, wenn sie durch die Retina, das Pigmentepithel und die Aderhaut eingesetzt wurde, so fest, dass sie ohne Beeinträchtigungen des Gewebes nicht wieder entfernt werden können. Von der Planfläche 11 geht ein zylindrischer Schaft 12 weiter in Richtung der in Figur 1 sichtbaren Oberfläche der Retina. Der Schaft 12 ist hohl und umgibt eine darin gelagerte Achse 13, die mit dem Hebel verbunden ist. Die Drehbarkeit der Achse 13 ermöglicht die Verschwenkung des Hebels von der Verankexungsstellung 4 in die Freigabestellung 5 in Richtung des Doppelpfeils 6.

Zur Implantation der Mikrokontaktfolie 2 kann diese entweder wie mit den bekannten micro tacks eingesetzt und dann mit den Verankerungsvorrichtungen 3 unmittelbar angeheftet werden, wobei die Verankerungsvorrichtungen 3 wie in Figur 1 gezeigt neben der Mikrokontaktfolie eingesetzt werden und der Hebel in der Verankerungsstellung 4 ist. Alternativ können zuerst die Verankerungsvorrichtungen 4 an die gewünschte Stelle eingesetzt werden, wobei sich die Hebel in der Freigabeposition 5 befinden. Sodann kann zwischen die Verankerungsvorrichtungen 3 die Mikrokontaktfolie 2 eingesetzt werden und die Hebel in die Verankerungsposition 4 verschwenkt werden. In beiden Fällen ist das Implantat am gewünschten Implantationsort gesichert.

Sollte das Implantat defekt werden oder gegen ein anderes Implantat ausgetauscht werden müssen, so können die Hebel in die Freigabeposition 5 verschwenkt werden, das Implantat kann ohne Beeinträchtigung des Gewebes entfernt werden und ein neues Implantat kann eingesetzt werden. Dies ist insbesondere deshalb von Vorteil, weil das mit dem Implantat kontaktierte Gewebe allenfalls geringfügig mechanisch belastet werden muss. Eine Beschädigung des Gewebes, wie sie bei den herkömmlichen micro tacks unvermeidbar ist, ist ausgeschlossen.

Es sind auch andere, nicht veranschaulichte Ausführungsformen der vorliegenden Erfindung denkbar. So kann beispielsweise der Hebel der in Figur 1 und 2 veranschaulichten Verankerungsvorrichtung nicht parallel zur Oberfläche des Implantats verschwenkt werden, sondern vertikal. Es ist auch denkbar, nicht eine Lagerung der Achse 13 in dem Schaft 12 vorzusehen, sondern eine Drehung der Verankerungsvorrichtung insgesamt zu ermöglichen, um den Hebel von der Verankerungsstellung 4 in die Freigabestellung 5 zu verschwenken. In jedem Falle wird die Entfernung einer bereits implantierten Mikrokontaktfolie durch die erfindungsgemäßen Verankerungsvorrichtungen wesentlich gewebeschonender sein. Zum Vorteil des Implantatträgers wird deshalb eher ein Austausch des Implantats vorgenommen werden können als bei den bislang bekannten Verankerungsvorrichtungen.

Die vorliegende Erfindung schafft also eine lösbare Befestigung bzw. Halterung von Mikrokontaktfolien, insbesondere an Retinagewebe. Zu diesem Zweck werden die Mikronägel entweder am Rand der Kontaktfolie ohne Durchführung durch ein Loch in der Folie positioniert, so dass für eine Re-Explantation die sehr flexible Folie durch Biegung unter den mit der Folie abschnittsweise überlappenden Verankerungsköpfen der Mikronägel hervorgezogen wird; alternativ werden die Verankerungsköpfe beweglich, aber mit Eigenhaftung in der eigentlichen Verankerungsstruktur befestigt (oder die als einseitig herausragende Struktur des Kopfes ist fest mit der Verankerungsstruktur verbunden und wird insgesamt in der Netzhaut gedreht) und können zur Re-Explantation gedreht (s. Abb. 2) oder anderweitig herausgezogen werden, ohne die Position der die Netzhaut durchdringenden Verankerungsstruktur wesentlich zu verändern.

## Patentansprüche

1. Vorrichtung zur Verankerung von implantierbaren Mikrokontaktfolien (2) als Neuroimplantat für Retina lmplants, umfassend eine als Mikronagel ausgebildete Verankerungsstruktur (3), wobei ein Verankerungskopf (4) die Verankerungsstruktur (3) zumindest abschnittsweise die implantierte Mikrokontaktfolie (2) überragen kann, **dadurch gekennzeichnet, dass** der Verankerungskopf (4) eine Verankerungsstellung und eine Freigabestellung bezüglich der Mikrokontaktfolie (2) aufweist.

2. Vorrichtung nach Anspruch 1, **dadurch gekennzeichnet, dass** die Verankerungsstruktur (3) durch Retina, Pigmentepithel und Aderhaut einsetzbar ist.

3. Vorrichtung nach Anspruch 1 oder 2, **dadurch gekennzeichnet, dass** der Verankerungskopf (4) als gegenüber der Verankerungsstruktur (3) verschwenkbar ausgebildet ist.

4. Vorrichtung nach einem der Ansprüche 1 bis 3, **dadurch gekennzeichnet, dass** der Verankerungskopf (4) die Mikrokontaktfolie (2) zum Zwecke der Befestigung überragt und dass der Verankerungskopf (4) zum Zwecke des Lösens der Mikrokontaktfolie (2) drehbar, schwenkbar, klappbar oder herausziehbar ist.

5. Vorrichtung nach einem der Ansprüche 1 bis 3, **dadurch gekennzeichnet, dass** der Verankerungskopf mit der Verankerungsstruktur lösbar verbindbar ist.

6. Vorrichtung nach einem der Ansprüche 1 bis 3, **dadurch gekennzeichnet, daß** der Verankerungskopf einen verschwenkbaren Hebel (4) umfaßt.

7. Vorrichtung nach Anspruch 6, **dadurch gekennzeichnet, daß** der verschwenkbare Hebel (4) mittels einer drehbaren Achse (13) gegenüber der Verankerungsvorrichtung von der Freigabestellung in die Verankerungsstellung bewegbar ist.

## Claims

1. Device for anchoring implantable micro-contact foils (2) for use as neuro-implants for retina implants, comprising an anchoring structure (3) in the form of a micro-tack, which anchoring structure (3) is able to extend above at least certain portions of the implanted micro-contact foil (2) by means of an anchoring head (4), **characterised in that** the anchoring head (4) has an anchoring position and a release position with respect to the micro-contact foil (2).

2. Device as claimed in claim 1, **characterised in that** the anchoring structure (3) can be inserted through the retina, pigmented epithelium and choroid membrane.

3. Device as claimed in claim 1 or 2, **characterised in that** the anchoring head (4) is designed to be pivotable relative to the anchoring structure (3).

4. Device as claimed in one of claims 1 to 3, **characterised in that** the anchoring head (4) extends above the micro-contact foil (2) for fixing purposes and the anchoring head (4) can be rotated, pivoted, folded or pulled out for the purpose of releasing the micro-contact foil (2).

5. Device as claimed in one of claims 1 to 3, **characterised in that** the anchoring head is releasably connected to the anchoring structure.

6. Device as claimed in one of claims 1 to 3, **characterised in that** the anchoring head has a pivotable lever (4).

7. Device as claimed in claim 6, **characterised in that** the pivotable lever (4) can be displaced relative to the anchoring device from the release position into the anchoring position by means of a rotatable shaft (13)..

## Revendications

1. Dispositif destiné à l'ancrage de feuilles de microcontact implantables (2) comme implant neurologique pour des implants rétiniens comprenant une structure d'ancrage (3) conçue comme une micro-cheville, dans lequel une tête d'ancrage (4) de la structure d'ancrage (3) peut surplomber la feuille de microcontact implantée (2) au moins par tronçons, **caractérisé en ce que** la tête d'ancrage (4) présente une position d'ancrage et une position de libération par rapport à la feuille de microcontact (2).

2. Dispositif selon la revendication 1, **caractérisé en ce que** la structure d'ancrage (3) peut être insérée à travers la rétine, l'épithélium pigmentaire et la choroïde.

3. Dispositif selon la revendication 1 ou 2, **caractérisé en ce que** la tête d'ancrage (4) est conçue pour pouvoir pivoter par rapport à la structure d'ancrage (3).

4. Dispositif selon l'une quelconque des revendications 1 à 3, **caractérisé en ce que** la tête d'ancrage (4) surplombe la feuille de microcontact (2) pour la fixation et **en ce que** la tête d'ancrage (4) peut être tournée, pivotée, repliée ou extraite pour détacher la feuille de microcontact (2).

5. Dispositif selon l'une quelconque des revendications 1 à 3, **caractérisé en ce que** la tête d'ancrage peut être reliée de manière détachable avec la structure d'ancrage.

6. Dispositif selon l'une quelconque des revendications 1 à 3, **caractérisé en ce que** la tête d'ancrage comprend un levier pivotant (4).

7. Dispositif selon la revendication 6, **caractérisé en ce que** le levier pivotant (4) est déplaçable entre la position de libération et la position d'ancrage par rapport au dispositif d'ancrage à l'aide d'un pivot (13).
